Europäisches Patentamt

(19) European Patent Office

Office européen des brevets

(11) Publication number: **0 064 747**
**A2**

## EUROPEAN PATENT APPLICATION

(21) Application number: **82104001.1**

(22) Date of filing: **07.05.82**

(51) Int. Cl.³: **A 61 M 31/00**
**A 61 F 13/20, B 65 D 75/30**

(30) Priority: **08.05.81 ES 258186**
**08.05.81 ES 258187**
**04.11.81 ES 261230**
**04.11.81 ES 261231**

(43) Date of publication of application:
**17.11.82 Bulletin 82/46**

(84) Designated Contracting States:
**AT BE CH DE FR GB IT LI LU NL SE**

(71) Applicant: **Asuncion S. Estadella, Maria**
**Bajada de la Gloria, 47**
**Barcelona(ES)**

(72) Inventor: **Asuncion S. Estadella, Maria**
**Bajada de la Gloria, 47**
**Barcelona(ES)**

(74) Representative: **Berg, Wilhelm, Dr. et al,**
**Dr. Berg, Dipl.-Ing. Stapf, Dipl.-Ing. Schwabe, Dr. Dr.**
**Sandmair Mauerkircherstrasse 45**
**D-8000 München 80(DE)**

(54) Wrapping for containing and introducing an insertable object into sphincter of human and animal bodies.

(57) The present invention refers to a wrapping which is designed to contain an object which is insertable into the sphincters of a human or an animal body. Preferably this body is a suppository or else a tampon for use during feminine menstrual periods. The wrapping as defined in the invention consists of a large bag which dimensionally far exceeds the dimensions of the object itself and a lodging situated in the interior of the said bag for containing the object. This wrapping provides an ample protection for the object contained therein and is such that it permits contact between the object and the hand or fingers of the person applying it to be avoided. Also, and of great importance, it allows the object to be administered to the sphincter in such a way that it protects the hands or the fingers of the person administering it from coming into direct contact with the zone of the body, as for example the anal or vaginal sphincter, into which the object is being inserted.

FIG.1

DR. W. BERG DIPL.-ING. O. STAPF
DIPL.-ING. SCHWABE DR.DR.SANDMAIR
PATENTANWÄLTE
8 MÜNCHEN 80, MAUERKIRCHERSTR. 45

0064747

Attorney's file: 50 256          7th May 1982

Maria Asuncion S. Estadella

Barcelona / Spain

- 1 -

## Wrapping for containing and introducing an insertable object into sphincters of human and animal bodies.

Treatment in both human and veterinary medicine, both from the therapeutic aspect as well as for hygienic and for prophylactic purposes, on numerous occasions has recourse to the introduction of objects into the interior of the body being treated in order to achieve the desired objective. One of the best known examples, though not the only one, is the suppository, there being two basic types of these; suppositories for insertion into the anal sphincter and suppositories that are inserted into the vaginal sphincter. A further well known example of an insertable object which is introduced into the body through the sphincter, and one becoming continually of wider use, is the tampon for feminine use during the menstruation period.

The use of such objects, be they suppositories, vaginal tampons or others of a similar nature presents however two important difficulties; The first of these is the certainty that the object will remain in its ideal condition of sterility or asepsis until after it has penetrated the body. In fact on any such occasion that the objects may have come into contact with the fingers or the hand of someone, there arises a serious risk of contamination and consequently a prime objective proposed by the invention is to permit that the operation of applying these objects to the body of a person or of an animal through one of its sphincters may take place without requiring in any way that it be in contact directly with the hand of the person who
who is carrying out the insertion. Furthermore there is another
useful advantage of note in the case of the application of suppositories insofar as there is no contact between the fingers
and the object itself, such as the suppository, thus ensuring
that the latter does not absorb heat from the human body and
consequent phenomena of undesirable softening.

The other difficulty, equally important, lies in ensuring with absolute certainty that the hand or the fingers of the person applying the object do not enter into contact with the zone of
insertion, that is to say the sphincter or the area immediately
surrounding the sphincter, in view of the fact that in such a case
the person in question would be subject to the risk of infection
from pathogenic organisms. The invention offers equally a very
satisfactory solution to this difficulty.

There are of course known devices and apparatus
having the same functional objectives as those described but in
general they are of a relatively complex structure and hence
have a high cost of manufacture. On the contrary the wrapping
subject of the invention is of a very simple structure and hence
the cost of its manufacture is low.

Furthermore not all the known devices serve at the
same time as a wrapping for the object, this being another of
the advantages arising from the invention. In point of fact the
wrapping, according to the invention serves for the storage,
the transport and the preservation, under suitable conditions, of
the object which is wrapped.

And finally, it should be pointed out that some of the
known devices and apparatus require that the person administering the object with their aid be other than the person to whom it is being administered, such that in the case of treatment of humans, it is limited to its use in such places as clinics and infirmaries. On the other hand, another of the advantages arising from the invention of this wrapping consists in the fact that any adequately competent person can self-administer it directly and on their own. It is evident that this characteristic is particularly desirable in the case of suppositories and tampons for feminine periodic use.

## DESCRIPTION OF THE INVENTION

The wrapping for containing and introducing an insertable object into sphincters of human and animal bodies, in accordance with the invention comprises a large bag having in one of its extremities an internal lodging is which is housed the relevant object, being for preference a suppository or a tampon for feminine periods. Both the large bag as well as the lodging for the object are made of some impermeable, flexible, sheet material, which by reason of its composition, can have parts of its surface mutually joined together by means of lines of welding which can be carried out with mechanical means or by heat. A plastic material is particularly suitable for the constituant material as it possesses all the said characteristics. In plastic material the ability to join the surfaces that need to be joined very simply by means of heat welding processes, along the lines of weld, is especially important. Also its impermeability is very high.

The large bag has a dual function; on the one hand it protects the object from external ambient conditions, while complementing this function with that of envelopping and protecting the object specifically by means of the lodging in which the object is housed. On the other hand, when carrying out the   -----

procedure of inserting the object into the body under treatment, having initially torn along the seam, weld line or other similar provision which has provided the isolation of the said object, the large bag is stretched over so as to cover the part of the hand or the fingers that otherwise would come into direct contact with the skin. in the area of the sphincter of the recipient, person or animal, of the object which is to be inserted.

According to some of the preferred embodiments the large bag is bell shaped, with the inner lodging in which is housed the object to be inserted into the body situated in the narrower extremity of the said large bag. This bell shape form is particularly useful so as to allow it to accomodate itself adequately to the anatomical shapes of the sphincter in the course of the application of the object in its interior such that the covering of the hand and/ or the fingers of the person applying it, is complete.

According to an embodiment which incorporates the characteristics described above the interior lodging which contains the object consists of an open compartment which is formed from the same sheet of material of which the large bag is formed. As this compartment is open, protection of the object is provided for by means of the closure of the edge of the wider extremity of the said large bag. The delimitation of the internal lodging is provided by lines of weld.

The embodiment described in the immediately preceeding paragraph can have disadvantages in the case of objects made from heat sensitive materials inasmuch as if, for any reason the mass of the object dissolves it will be dispersed in the inside of the large bag in an uncontrolled manner and without the possibility of recovery. Suppositories are particularly subject to this disadvantage. On the other hand menstrual period tampons for feminine use do not lose their efficacity in use in the case of the above embodiment subject of this observation. With the view to obviating the above mentioned defect the wrapping according to the invention has another characteristic in that the internal lodging is formed into a closed housing taking the form of an appendix and is shaped from the same sheet of material which makes up the large bag by means of carrying out a succession of bends or folds. Lines of weld and/or seals partially delimit this housing.

In accordance with the other characteristic of the wrapping object of this invention the internal lodging contain- the object which is insertable into the body consists of a closed housing which is formed from the same sheet of material as the large bag. The whole extension of this housing is formed at the expense of the large bag which in consequence is correspondingly reduced in its length. Lines of weld delimit totally or partially the extension of the said housing.

Finally and according to another characteristic of the wrapping which is disclosed in the invention, the internal lodging containing the object to be inserted into the body consists of a closed small bag the forming of which is carried out independently from the forming of the large bag. Thus this small independent bag can be made at a stage previous to the stage of finalizing of the wrapping. Subsequently this small bag is joined to the large bag by welding, at its narrower extremity, it being internally adapted thereto, forming an appendix.

## BRIEF DESCRIPTION OF THE DRAWINGS

On the four sheets accompanying this present specification are shown various embodiments of the wrapping according to the invention. In each of the said embodiments it has been   ----

assumed that the flexible and impermeable sheet material of which the wrapping is made is either transparent or translucent such that the whole structure of the wrapping may be seen through it. This, apart from facilitating the illustrations accompanying this specification, offers a practical advantage in the commercial products which are presented to the. public, principally for people who are utilizing for the first time or who utilize rarely the wrapping according to the invention. However this is not stricly necessary and it is understood that the said material can also be opaque.

FIGURE 1, illustrates an embodiment shown in elevation and presenting a general view.

FIGURE 2, offers a perspective view illustrating the method of using the wrapping shown in the same embodiment, this, at the stage of inserting the object into the person or the animal under treatment.

FIGURES 3 to 6, show another embodiment of a wrapping according to the invention.  ·

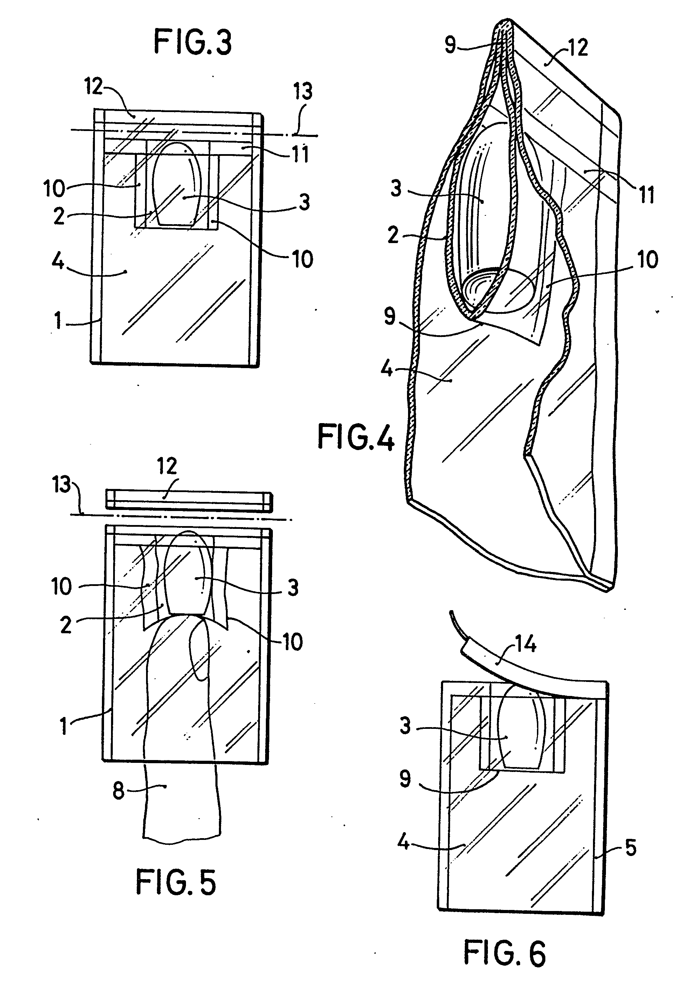

FIGURE 3, is a general view.

FIGURE 4, is a perspective view, partially, but in part cut away to show clearly the structure of the wrapping.

FIGURE 5, shows the method of proceeding to eject the object to the exterior.

FIGURE 6, illustrates the cut which must be made prior to applying so as to allow a free passage of the object to the exterior of the wrapping.

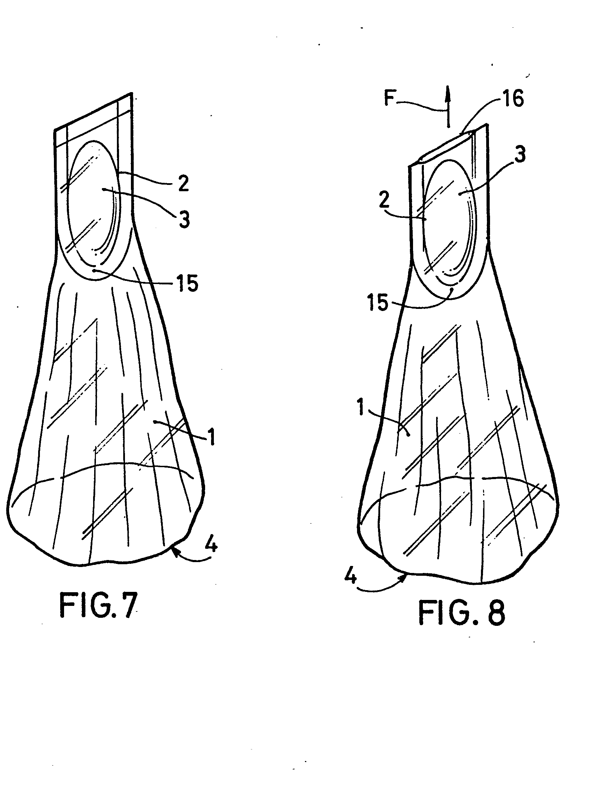

FIGURES 7 and 8, refer to another embodiment, both of them showing a perspective view. In the first case the said wrapping is whole . In the second case the wrapping is shown having been cut in such a way that the object to be inserted into the sphincter has a free passage towards the exterior.

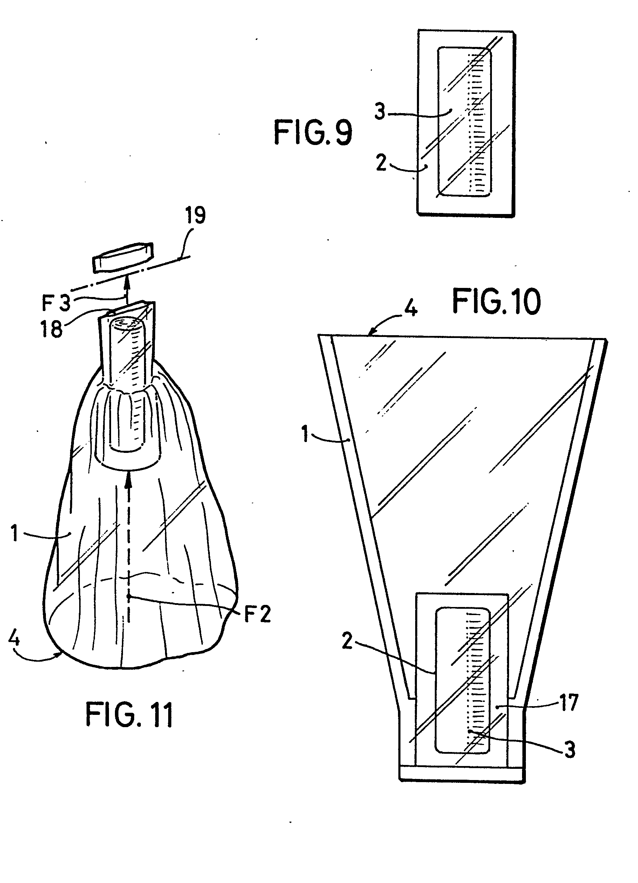

FIGURES 9, 10 and 11, shows another embodiment.

FIGURE 9, shows separately one of the elements of which the wrapping is comprised.

FIGURE 10, shows a general view of the complete wrapping.

FIGURE 11, is a perspective view which illustrates graphically the procedure of utilization of this wrapping during the operation of applying the object which is contained therein.

## DETAILED DESCRIPTION OF THE EMBODIMENTS

In all the embodiments shown in the drawings and referred to previously the large bag is numbered 1, the internal lodging is numbered 2, the object is shown against number 3, in each case it being housed in the lodging 2, this object being that which is to be protected by the wrapping and destined to be inserted into a body, human or animal via one of its sphincters. In some cases the drawings show a suppository as an illustration of the aforementioned object, in other cases a feminine menstrual period tampon is shown. It is understood that the examples referred to are simply those which relate the the more usual and representative applications of the invention. However such variations in no way affect the essentiality of the invention and may extend to other objects which have an equal or similar destination. For this reason the reference No. 3 in all cases relates thereto. Also so as to standardize the identification of similar components the interior of the large bag 1 carries the reference No. 4 in all the examples.

The structural simplicity of the wrapping according to the invention is such that the drawings are for the most part self explanatory.

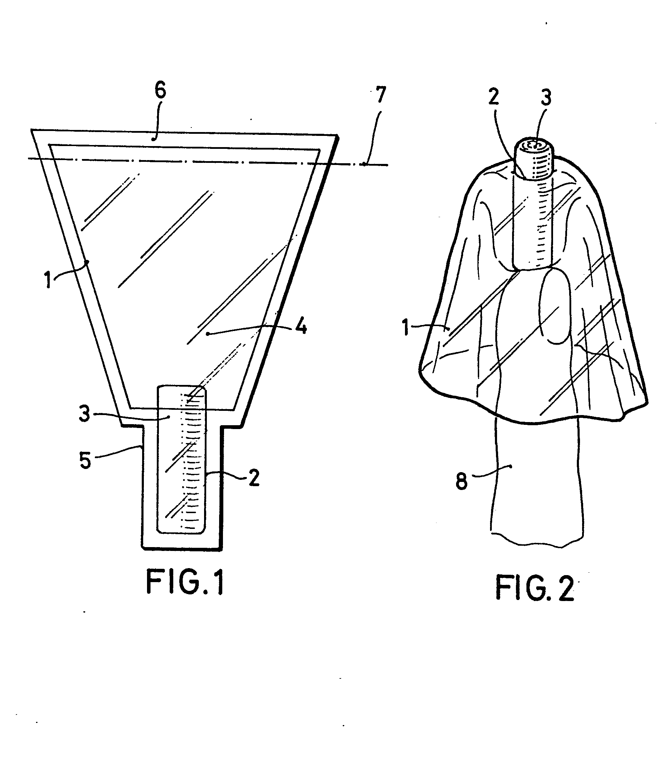

In the example shown in Figures 1 and 2 the internal lodging 2 is formed by lines of weld 5 which leave its front extremity with its orientation towards the interior 4 of the large bag 1, open, the bag being bell-shaped. The object 3 is thus free as to its frontal aspect. However its protection with respect to its aseptic conditions remaining satisfactory is maintained by means of the closure line 6 provided at the wider extremity of the large bag 1. To carry out the application of the object 3 it is sufficient to tear or cut approximately along the direction of the line 7, which line may have been indicated, drawn or initiated by means of a die. FIGURE 2 shows how the object 3 is made to eject by means of pushing with a finger 8. Thus it is by no means necessary that the object 3 be touched directly by the fingers. Also it may be seen how the large bag 1 is turned inside out so as to take the shape of a funnel. This provides ample protection for the operating finger 8 as well as for the adjacent fingers and the surrounding area of the hand, this being easily deduced though not shown in the drawing. In this manner all possibility of direct contact with the sphincter and the surrounding zone is avoided.

FIGURES 3 to 6 can be seen embodiment examples in which the large bag is of rectangular shape and in the inside 4, of which, is provided the internal lodging 2 , taking the form of closed housing forming an appendix or boss. This housing is formed from the same flexible impermeable sheet of material forming the structure of the large bag 1, this being achieved by means of successive folds or bends 9. The perimeter of the lodging 2 is delimited by lines of weld 10 while other lines of weld 11 provide the absolute separation between the inside of the bag 1 and the opposite from which the object 2 must be ejected, thus protecting the fingers 8 of the person applying it from entering into direct contact with the sphincter or surrounding areas.

The closure of the internal lodging 2 can be provide for in two ways. In FIGURES 3,4 and 5 this is carried out by providing the line of weld, such that by cutting along the direction of the line 13 the object is ready for applying as is clearly shown in FIGURE 5. This line 13 may be indicated, drawn or stamped on the surface of the wrapping. According to the other method of closure there is provided a seal 14 which obturates the bag 1 at the extremity at which is located the internal lodging 2 such that the previously mentioned line of weld 12 can be dispensed with. By the action of opening the said seal 14, the object 3 is able to eject to the exterior by pushing from the inside of the bag 1, in the same manner as is illustrated in the FIGURE 5.

FIGURES 7 and 8 show another embodiment of the wrapping according to the invention. The large bag 1 is bell-shaped with the internal lodging 2 in which is situated the object 3, being a completely closed housing shaped from the same sheet of flexible material of which is formed the bag 1. The delimitation of the internal lodging 2 is provided by lines of weld 15. This embodiment is of very great simplicity. To carry out the application of the object 3 a cut or tear 16 is made leaving the path of ejection free as shown by the arrow F. In this case the the pushing action on the object 3 is carried out by placing the finger (not shown in these two FIGURES 7 and 8) in the inside 4 of the bag 1. The cut or the tear 16 can be made either by the use of a cutting tool or instrument or with the aid of a stamped or weakened line provided so as to allow its breaking by direct simple manual action.

FIGURES 9, 10 and 11 show graphically another example of the embodiment according to the invention. --------

In this case the internal lodging 2 in which is housed the object 3, consists of a small closed bag of which the filling with the object 3 can be carried out previous to the completion of the whole structure of the wrapping. This is not considered to be a necessary condition but solely one possibility derived from this means of structure formed from two distinct components, the large bag 1, and the small bag 2 which, of course, and in accordance with the fundamental characteristics of the invention, are firmly united once the wrapping has acquired its final shape. In fact, FIGURE 9 shows the simple structure of the small bag 2 which forms the internal lodging for the object 3. Also in FIGURE 10 can be seen the complete wrapping in which the small bag 2 has been joined on to the large bag 1 by means of lines of weld 17.. The small bag 2 projects from the bottom of the interior 4 of the bag 1 forming an appendix thereto. The application of the object 3 is by previously having torn or broken open the small bag 2 and then turning the bag 1 inside out leaving the former exposed. FIGURE 11 shows this method of procedure. The cut or tear 18 is made along the line 19 which may have been indicated graphically or even stamped or weakened so as to facilitate this action for the user. Thus the ejection path of the object 3 is left clear and all that is required is to push with a finger as is shown by the arrow F2, that is to say by introducing the finger into the bell-shape that has been formed on turning the bag 1 inside out, forcing it towards the exterior as shown by the arrow F3 and thus achieving the insertion of the object into the interior of the sphincter in a manner which is hygienic, simple and comfortable.

## C LAIM S

1. Wrapping for containing and introducing an insertable object into sphincters of human and animal bodies, characterized insofar as it consists of a large bag having at one of its extremities an internal lodging in which is housed the relevant object, this being for preference a suppository or a tampon for feminine menstrual periods, the said large bag and the said internal lodging being made from flexible sheet material, capable of having parts of its area mutually joined together by means of lines of welding which welding operation may be carried out by mechanical means or by the application of heat.

2. Wrapping as defined in claim 1, which is characterized by the large bag being bell-shaped and by the internal lodging containing the object which is insertable into the body being situated at the narrower extremity of the said bag.

3. Wrapping as defined in claims 1 and 2, which is characterized by the internal lodging containing the object which is insertable into the body being in the form of an open housing and formed from the same sheet of material from which the large bag is made, this said housing being totally or partially delimited by lines of welding, the wider extremity of the bag being closed.

4. Wrapping as defined in claim 1, which is characterized by the internal lodging containing the object which is insertable into the body consisting of a closed housing which takes the form of an appendix this same being formed from the same sheet of material which forms the large bag, by the action of successive folding and being partially delimited by lines of welding and/or sealing.

5.  Wrapping as defined in claims 1 and 2, which is characterized inasmuch as the internal lodging containing the object which is insertable into the body consists of a closed housing formed from the same sheet of material which forms the large bag, the said housing being totally or partially delimited by lines of welding.

6.  Wrapping as defined in claims 1 and 2, which is characterized insofar as the internal lodging containing the body which is insertable into the body consists of a small closed bag which originally independent of the large bag, is joined to it by welding giving it the form of an appendix.

========================

FIG.1

FIG.2

**FIG.3**

**FIG.4**

**FIG.5**

**FIG.6**

0064747

FIG.7

FIG. 8

FIG.9

FIG.10

FIG.11